# EUROPEAN PATENT APPLICATION

(11) **EP 4 427 747 A1**
(43) Date of publication of application: **11.09.2024**
(21) Application number: 22889288.1
(22) Date of filing: 01.11.2022
(51) Int. Cl.: A61K 31/437, A61K 31/5025, A61P 35/00

(54) **SOLID DISPERSION, PREPARATION METHOD THEREFOR, AND SOLID FORMULATION CONTAINING SAME**

(30) Priority: 05.11.2021 CN 202111308836
(71) Applicant: Haihe Biopharma Co., Ltd., Shanghai 201203 (CN)
(72) Inventor: GAN, Yong, Shanghai 201203 (CN); ZHU, Miao, Shanghai 201203 (CN); MA, Yuanhui, Shanghai 201203 (CN); LIU, Lei, Shanghai 201203 (CN); GUO, Shiyan, Shanghai 201203 (CN); SHEN, Jingkang, Shanghai 201203 (CN); GENG, Meiyu, Shanghai 201203 (CN); GAO, Li, Shanghai 201203 (CN); XIONG, Bing, Shanghai 201203 (CN)
(74) Representative: Mewburn Ellis LLP
(86) International application number: PCT/CN2022/129074
(87) International publication number: WO 2023/078265

(57) **Abstract**

A solid dispersion, a preparation method therefor, and a solid formulation comprising same. The solid dispersion comprises compound A and a pharmaceutically acceptable matrix polymer, wherein the pharmaceutically acceptable matrix polymer includes an enteric high-molecular polymer and a non-enteric high-molecular polymer, and the compound A is 1-{(6-[(1-methyl)-4-pyrazolyl]-imidazo[1,2-a]pyridine)-3-sulfonyl} -6-[(1 -methyl)-4-pyrazolyl]-1 -hydro-pyrazolo[4,3-b]pyridine. The solid dispersion can significantly improve the solubility and dissolution stability of the compound A, prolong the supersaturation maintenance time of a drug, and further improve the bioavailability of the drug. The in vivo bioavailability of a solid formulation prepared from the solid dispersion meets the requirement of oral administration of the compound A.

## Description

The present application claims priority for Chinese patent application 2021113088364 filed on November 5, 2021. The present application cites the full text of the aforementioned Chinese patent application.

### TECHNICAL FIELD

The present invention belongs to the field of drug formulations, and specifically relates to a solid dispersion, a preparation method thereof, and a solid drug formulation comprising the same, as well as an use of the solid dispersion in the preparation of a drug for the prevention and/or treatment of protein tyrosine kinase disorder-related disease and tumor.

### BACKGROUND

Patent CN104230922A disclosed compound A (1-{(6-[(1-methyl)-4-pyrazolyl]-imidazo [1,2-a]pyridine)-3 -sulfonyl} -6- [(1 -methyl)-4-pyrazolyl] -1 -hydro-pyrazolo [4,3 - b]pyridine), lists the use of compound A and pharmaceutically acceptable salts thereof in preparation of a drug for the prevention or treatment of abnormal cell proliferation, morphological change, and hyperkinesia related to protein tyrosine kinase disorder in an organism, as well as diseases related to angiogenesis or cancer metastasis, especially use in the preparation of a drug as a C-Met inhibitor.

Overexpression of C-Met can be seen in human liver cancer, cholangiocarcinoma, pancreatic cancer, lung cancer, thyroid cancer, pleural stromal tumor, especially in metastatic tumors. Its effects may include affecting adhesion between tumor cells, promoting extracellular matrix degradation, inducing angiogenesis, and promoting cell proliferation. All of these indicate that C-Met is an important target for treating tumors. Compound A is a highly selective C-Met inhibitor, and its inhibitory effect on C-Met and in vitro and in vivo anti-tumor activity are superior to similar drugs INCB28060 (CAS number: 1029712-80-8) in existing clinical applications. Compound A has strong activity, minimal toxic side effects, and good prospects.

Further research on compound A revealed that its equilibrium solubility was below 1.0 µg/mL in a buffered saline solution at pH 1.2-7.4, and it is a water-insoluble drug. The results of animal level research show that the in vivo bioavailability of compound A after direct administration is less than 1%, and the in vivo absorption is poor, making it unable to effectively exert therapeutic effects. Therefore, it is necessary to increase solubility and improve oral absorption before use.

The inventors attempted the commonly used conventional solubilization and absorption methods and found that there were certain problems with compound A: (1) they attempted to make compound A into a salt, but found no significant improvement in its solubility; (2) they attempted to make compound A into different crystal forms, and found no significant difference in solubility among different crystal forms; (3) they attempted to micronize compound A to enhance solubility and absorption, and found that its bioavailability was only 3.3%, making it unsuitable for oral administration; and (4) they attempted to prepare a solubilizing solution of compound A using a solubilizer, but compound A has a melting point greater than 250°C and a strong tendency towards crystallization. The solution exhibits crystallization after being placed, resulting in poor long-term stability and inability to dissolve after crystallization, which cannot solve the problem of poor drug absorption.

### SUMMARY

The technical problem to be solved by the present invention is to overcome the shortcomings of poor solubility of compound A in water and low in vivo bioavailability in the prior art, and to provide a solid dispersion, a preparation method thereof, and a solid formulation comprising same.

Compound A in the solid dispersion of the present invention has a high solubility in simulated intestinal fluid. Furthermore, the solid dispersion of the present invention can significantly improve the solubility and dissolution stability of compound A, prevent drug precipitation, prolong the oversaturation maintenance time of the drug, thereby improve the bioavailability of the drug. The solid formulation of the present invention has high bioavailability.

The present invention effectively controls the decomposition of components in the dispersion by improving the preparation process of the solid dispersion, especially the degradation of matrix polymer materials, thereby reducing the impurity content of the dispersion. The present invention also greatly improves the compressibility of the tablets obtained from the solid dispersion system by optimizing the solid dispersion crushing process and the mixing process of the solid dispersion powder, avoiding situations such as low tablet hardness, poor fragility, and severe transportation powder detachment.

On the one hand, the present invention provides a solid dispersion comprising compound A and a pharmaceutically acceptable matrix polymer, wherein the pharmaceutically acceptable matrix polymer includes an enteric high molecular polymer and a non-enteric high molecular polymer, and the compound A is 1-{(6-[(1-methyl)-4-pyrazolyl]-imidazo[1,2-a]pyridine)-3-sulfonyl}-6-[(1-methyl-4-pyrazolyl]-1-hydro-pyrazolo[4,3-b]pyridine, with a weight ratio of the compound A to the pharmaceutically acceptable matrix polymer of 1:3-1:35.

Furthermore, the solid dispersion also optionally comprises one, two, or three of a flow aid, a plasticizer, and a surfactant.

In a specific embodiment of the present invention, preferably, the enteric high molecular polymer is selected from one or more of hydroxypropyl methylcellulose phthalate (HPMCP), hydroxypropyl methylcellulose acetate succinate (HPMCAS), polymethacrylate, polyvinyl acetate phthalate (PVAP), cellulose acetate phthalate, and cellulose acetate succinate; and more preferably, the enteric high molecular polymer is hydroxypropyl methyl cellulose phthalate and/or hydroxypropyl methylcellulose acetate succinate.

In a specific embodiment of the present invention, preferably, the non-enteric high molecular polymer is selected from one or more of polyvinyl caprolactam-polyvinyl acetate-polyethylene glycol graft copolymer (Soluplus), copovidone (i.e. N-vinylpyrrolidone/vinyl acetate copolymer, PVP/VA), povidone (i.e. polyvinylpyrrolidone, PVP), polyvinyl alcohol, 2-hydroxy-β-cyclodextrin (HPBCD), hydroxypropyl methylcellulose (HPMC), and hydroxypropyl cellulose (HPC); and more preferably, the non-enteric high molecular polymer is selected from one or more of copovidone, polyvinyl alcohol, povidone, and hydroxypropyl methylcellulose.

In a specific embodiment of the present invention, the pharmaceutically acceptable matrix polymer comprises any combination of hydroxypropyl methylcellulose phthalate and povidone, hydroxypropyl methylcellulose phthalate and copovidone, hydroxypropyl methylcellulose acetate succinate and povidone, hydroxypropyl methylcellulose phthalate and hydroxypropyl methylcellulose, hydroxypropyl methylcellulose acetate succinate and polyvinyl alcohol, hydroxypropyl methylcellulose acetate succinate and copovidone, hydroxypropyl methylcellulose phthalate and polyvinyl alcohol, cellulose acetate phthalate and povidone, or cellulose acetate succinate and copovidone.

In a specific embodiment of the present invention, the weight ratio of the enteric high molecular polymer to the non-enteric high molecular polymer can be 2:1-10:1, preferably 2:1-6:1, for example, 2:1, 2.5:1, 3:1, 3.5:1, 4:1, 4.5:1, 5:1, or 6:1.

In a specific embodiment of the present invention, the weight ratio of compound A to the pharmaceutically acceptable matrix polymer can be 1:4-1:25, preferably 1:5-1:15, for example, 1:4, 1:5, 1:5.5, 1:6, 1:7.5, 1:8, 1:9, 1:10, 1:12, 1:15, or 1:21. According to the in vivo pK test, when the weight ratio of compound A to the pharmaceutically acceptable matrix polymer is 1:4-1:25, it can not only improve the solubility of the prepared solid dispersion, but also significantly increase the in vivo exposure dose of compound A.

In a specific embodiment of the present invention, the weight ratio of compound A to the enteric high molecular polymer can be 1:2-1:15, more preferably 1:3-1:10, for example, 1:3, 1:4, 1:4.5, 1:5, 1:6, 1:8, or 1:10.

In a specific embodiment of the present invention, the weight ratio of compound A to the non-enteric high molecular polymer can be 2:1-1:10, preferably 2:1-1:5, more preferably 1:1-1:5, or 1:2-1:5, for example, 2:1, 1:1, 1:1.5, 1:2, 1:2.5, 1:3, 1:5, 1:8, or 1:10.

In a specific embodiment of the present invention, the flow aid can be a conventional flow aid in the art, preferably selected from one or more of colloidal silica, animal fat, plant fat, and wax, for example, colloidal silica. The amount of the flow aid can be selected according to the conventional amount of a flow aid in the art. Preferably, the weight ratio of the flow aid to compound A is 1:1-1:100, preferably 1:4-1:50, for example, 1:6, 1:10, 1:15, 1:20, 1:30, 1:50, 1:80, or 1:100.

In a specific embodiment of the present invention, the presence of a plasticizer can improve the processability of the solid dispersion, and the plasticizer can be a conventional plasticizer in the art, preferably, the plasticizer is selected from one or more of acetyl tributyl citrate, acetyl triethyl citrate, benzyl benzoate, trichlorobutyl alcohol, dextrin, dibutyl phthalate, diethyl phthalate, dimethyl phthalate, glycerol, glycerol monostearate, polyoxyl-40-stearate, mannitol, mineral oil, lanolin alcohol, palmitic acid, polyethylene glycol, polyethylene glycol monostearate, polyvinyl acetate phthalate, propylene glycol, 2-pyrrolidone, sorbitol, stearic acid, triacetin, tributyl citrate, triethanolamine, and triethyl citrate; and more preferably, the plasticizer is a plasticizer with a low glass transition temperature, such as glyceride monostearate and/or polyoxyl-40-stearate.

The amount of the plasticizer can be selected according to the conventional amount of a plasticizer in the art. Preferably, the weight ratio of the plasticizer to compound A is 1:1 -1:20, preferably 1:1-1:5, for example, 1:1.5, 1:2, 1:2.5, 1:5, 1:10, 1:15, or 1:20.

In a specific embodiment of the present invention, the surfactant can further enhance the therapeutic potential of the solid dispersion of the present invention. The surfactant can be a conventional surfactant in the art, preferably selected from one or more of an anionic surfactant, a cationic surfactant, and a non-ionic surfactant.

The anionic surfactant is preferably sodium dodecyl sulfate (sodium lauryl sulfate) and/or docusate sodium. The cationic surfactant is preferably one or more of cetrimide, benzethonium chloride, cetylpyridinium chloride, and lauric acid. The non-ionic surfactant is preferably one or more of polyoxyethylene alkyl ether, polyoxyethylene sorbitan fatty acid ester (such as Tween 80, 60, 40, and 20), polyoxyethylene castor oil derivative (such as polyoxyl 40 hydrogenated castor oil (Cremophor RH40)), polyoxyethylene stearate, and polyoxyethylene polyoxypropylene ether block copolymer (such as Poloxamer). More preferably, the surfactant is one or more of sodium dodecyl sulfate, docusate sodium, lauric acid, polyoxyethylene alkyl ether, polyoxyethylene sorbitan fatty acid ester, polyoxyethylene castor oil derivative, Poloxamer, and polyoxyethylene stearate. Most preferably, the surfactant is sodium dodecyl sulfate and/or polyoxyethylene stearate.

The amount of the surfactant can be selected according to the conventional amount of a surfactant in the art, preferably, the weight ratio of the surfactant to compound A is 1:1-1:10, preferably 1:1-1:5, for example, 1:2.5, 1:3, 1:4, 1:5, 1:8, or 1:10.

In a specific embodiment of the present invention, the solid dispersion comprises compound A, a pharmaceutically acceptable matrix polymer, a flow aid, and a plasticizer, wherein the pharmaceutically acceptable matrix polymer includes an enteric high molecular polymer and a non-enteric high molecular polymer.

In a specific embodiment of the present invention, the solid dispersion is composed of compound A, a pharmaceutically acceptable matrix polymer, a flow aid, and a plasticizer, wherein the pharmaceutically acceptable matrix polymer includes an enteric high molecular polymer and a non-enteric high molecular polymer.

In a specific embodiment of the present invention, the solid dispersion comprises compound A, a pharmaceutically acceptable matrix polymer, a flow aid, a plasticizer, and a surfactant, wherein the pharmaceutically acceptable matrix polymer includes an enteric high molecular polymer and a non-enteric high molecular polymer.

In a specific embodiment of the present invention, the solid dispersion is composed of compound A, a pharmaceutically acceptable matrix polymer, a flow aid, a plasticizer, and a surfactant, wherein the pharmaceutically acceptable matrix polymer matrix polymer includes an enteric high molecular polymer and a non-enteric high molecular polymer.

In the present invention, when the pharmaceutically acceptable matrix polymer includes an enteric high molecular polymer and a non-enteric high molecular polymer, not only can the solubility of the solid dispersion be improved, but also the processability of the solid dispersion can be improved. On the basis of the prior art, the inventors attempted to use commonly used solid dispersion techniques to enhance the solubility and absorption of compound A. However, the research results found that the solid dispersion of compound A made by using a single matrix polymer with conventional methods has certain problems. For example, the solid dispersion of compound A made solely by using commonly used non-enteric matrix polymer copovidone has a dissolution rate of less than 90% in simulated intestinal fluid for 90 minutes, poor supersaturation stability, and a bioavailability of only 6.3% (Comparative Example 3 and Experimental Examples 2, 3, and 4); and the solid dispersion of compound A prepared solely using enteric matrix polymer hydroxypropyl methylcellulose phthalate can slightly increase the solubility of compound A, but as time goes on, the solubility decreases, indicating drug precipitation and poor stability in oversaturated state (Comparative Example 4), which is not conducive to improving drug absorption. In vivo research results show that its bioavailability is only 12.4% (Experimental Example 4).

The inventor also unexpectedly discovered that the solid dispersion of the present invention can form stable mixed micelles with an average particle size of 100-200 nm in simulated intestinal fluid, thereby significantly improving the solubility of a drug through the principle of micelle solubilization, further avoiding drug precipitation, prolonging the oversaturation maintenance time of a drug, and improving drug bioavailability. The solid dispersion of the present invention overcomes the disadvantage of easy precipitation of ordinary solid dispersion drugs.

On the other hand, the present invention also provides a preparation method for the solid dispersion as described earlier, including the following steps:
(1) mixing the various ingredients of the solid dispersion uniformly by melting or dissolution to obtain a uniform dispersion; and
(2) solidifying the uniform dispersion to obtain the solid dispersion.

In a specific embodiment of the present invention, the solidification can be solvent evaporation method or melt extrusion method, and the melt extrusion method is preferred.

The melt extrusion method (hot melt extrusion method) produces a uniform dispersion by applying heat and/or mechanical stress. Specifically, the melt extrusion method involves mixing and extruding a drug, for example, excipients such as compound A, a pharmaceutically acceptable matrix polymer, and a plasticizer in a molten state to form a solid dispersion. This method can disperse a drug (such as a crystalline drug) in an amorphous or molecular state in a carrier material (a pharmaceutically acceptable matrix polymer) after heating and melting, ultimately improving the solubility, dissolution rate, and oral bioavailability of an insoluble drug. The uniform dispersion obtained by the melt extrusion method is also known as a melt, and "melt" refers to a liquid or rubber-like state, wherein one component may be uniformly embedded in other components. Generally, one component melts and other components dissolve in the molten material, forming a melt. The formation of a melt typically involves the softening point of the pharmaceutically acceptable matrix polymer, and the preparation of the melt can occur through various methods. Mixing of components can be carried out before, during, or after the formation of the melt. For example, firstly, components are mixed, then heated, or mixed and heated simultaneously. Usually, active substances in a melt should be uniformly dispersed, and the melt should be in a paste or viscous state. Usually, the working temperature in the present invention will be determined by the type of an extruder or the construction type of the extruder used. The partial energy required for the melting, mixing, and dissolution of components in an extruder can be provided through a heating element. The friction and shear of a material in an extruder can also provide a large amount of energy to the mixture, helping to form a uniform melt of components. The extruded material can be achieved using a molding module of an extruder, and can be cut into blocks before or after solidifying. The extrusion temperature of the melt extrusion method is 70-250°C, preferably 80-230°C, and most preferably 120-210°C.

In the melt extrusion method, the production and extrusion of the melt can be carried out in conventional devices; and the devices are preferably an extruder or a kneader. The extruder can be a rod extruder, including a single screw extruder, a double screw extruder, or other multi screw extruders, preferably a double screw extruder, which can rotate clockwise or counterclockwise and is arbitrarily equipped with a kneading plate.

In a preferred embodiment of the present invention, the melt extrusion method includes:
(1a) mixing the various ingredients of the solid dispersion uniformly to obtain a powdered mixture; and
(2a) loading the powdered mixture into a hot melt extruder feeder, extruding, crushing, and screening to obtain the solid dispersion comprising the compound A.

In the melt extrusion method, the sleeve temperature of the melt extrusion equipment is 150-220°C, preferably 150-200°C, 150-180°C, 180-200°C, and more preferably 160-180°C. When the temperature is between 150-200°C, it can avoid the increase of impurities caused by the increase in temperature and the decrease in screw speed (increase in residence time).

In the melt extrusion method, the screw extrusion rotating speed of the melt extrusion equipment is 50-300 rpm, preferably 50-240 rpm, 50-180 rpm, 100-210 rpm, or 180-240 rpm.

In the melt extrusion method, the feeding speed is 10-100 rpm, preferably 50-100 rpm or 50-70 rpm.

In the melt extrusion method, when the pharmaceutically acceptable matrix polymer includes an enteric high molecular polymer hydroxypropyl methylcellulose phthalate or polyvinyl acetate phthalate, controlling the extrusion sleeve temperature, screw speed, and feeding speed can avoid an increase in impurity phthalic acid content.

Preferably, the content of phthalic acid in the solid dispersion does not exceed 6 wt% (calculated based on the total component of the solid dispersion); more preferably, the content of phthalic acid does not exceed 4.8 wt%.

In other preferred embodiments, the solvent evaporation method includes the following steps:
(1b) dissolving the various ingredients of the solid dispersion in a solvent to obtain a uniform dispersion; and
(2b) removing the solvent from the uniform dispersion to obtain the solid dispersion.

In step (1b), the solvent may be a conventional solvent in the art, preferably selected from one or more ketone solvents, halogenated alkane solvents, alcohol solvents, and water. The ketone solvent is preferably acetone. The alcohol solvents are preferably isopropanol, methanol, and/or ethanol. The halogenated alkane solvent is preferably chloroalkane, more preferably dichloromethane or trichloromethane. The solvent is selected from acetone, acetone/dichloromethane, methanol/dichloromethane, acetone/water, acetone/methanol, acetone/ethanol, dichloromethane/ethanol or ethanol/water, etc., wherein "/" represents a mixed solvent of the two.

The solvent removal method in step (2b) can be a conventional solvent removal method in the art, preferably rotary evaporation, vacuum decompression drying, spray drying, freeze-drying and film evaporation; alternatively, solvent removal can be achieved by low-temperature freezing followed by freeze-drying; and other techniques can also be used, such as solvent controlled precipitation, pH controlled precipitation, and low-temperature co-grinding.

On the other hand, the present invention also provides a solid formulation comprising the solid dispersion as described earlier and a medicinal additive.

In the solid formulation, the medicinal additive can be a conventional medicinal additive in the art, preferably comprising one or more of a flow aid, an adhesive, a disintegrant, a filler, a colorant, a pH regulator, a surfactant, a lubricant, a stabilizer (such as an antioxidant, a light stabilizer, a free radical scavenger, a stabilizer against microbial attacks), etc. The specific selection range and amount of the additive are conventional choices in the art.

In the solid formulation, the adhesive can be a conventional adhesive in the art, preferably selected from one or more of copovidone, povidone, methyl cellulose, ethyl cellulose, and hydroxypropyl cellulose.

In the solid formulation, the colorant can be a conventional colorant in the art, and the amount of the colorant can be a conventional amount in the art.

In the solid formulation, the disintegrant promotes rapid disintegration of the solid formulation in the stomach while maintaining the separation of released particles from each other. Preferably, the disintegrant comprises a cross-linked polymer, for example, croscarmellose sodium and/or cross-linked polyvinylpyrrolidone (i.e. crospovidone PVPP).

In the solid formulation, preferably, the filler is selected from one or more of lactose, sucrose, mannitol, calcium hydrogen phosphate, microcrystalline cellulose, starch, and isomaltose.

In the solid formulation, preferably, the adhesive is selected from one or more of povidone, copovidone, methyl cellulose, ethyl cellulose, and hydroxypropyl cellulose. Among them, povidone and copovidone, which serve as pharmaceutically acceptable matrix polymers, can also act as adhesives.

In the solid formulation, preferably, the lubricant is selected from one or more of polyethylene glycol (for example, molecular weight 1000-6000), magnesium stearate, calcium stearate, and sodium stearyl fumarate.

In the solid formulation, preferably, the pH regulator is a conventional pH regulator in the art, preferably citric acid.

The solid formulation further includes a film coating, which can improve taste and provide a refined appearance, for example, the film coating of tablets can aid in swallowable comfort. The film coating can be a conventional film coating in the art, and the film coating can be a moisture-proof coating. The film coating typically comprises polymerized film-forming materials, for example, hydroxypropyl methylcellulose, hydroxypropyl cellulose, and acrylic or methacrylate copolymer. In addition to polymeric film-forming materials, the film coating may also comprise a plasticizer such as polyethylene glycol, a surfactant such as Tween, an anti-adhesive agent such as talc powder, and an optional pigment such as titanium dioxide or iron oxide. These additives can account for approximately 0% to 20% of the total weight of the solid formulation.

In a specific embodiment of the present invention, preferably, the solid dispersion can be 60%-90% of the mass of the solid formulation.

In a specific embodiment of the present invention, preferably, the medicinal additive can be 15%-40% of the mass of the solid formulation.

On the other hand, the present invention also provides a preparation method for the aforementioned solid formulation, including the following steps: mixing the powder or particles of the aforementioned solid dispersion with medicinal additives to form a solid formulation.

Preferably, the powder or particles of the solid dispersion are prepared by crushing, milling, or grinding the solid dispersion.

In the process of preparing the solid formulation, the present invention also greatly improves the compressibility of the solid formulation tablets obtained from the product solid dispersion system by optimizing the solid dispersion crushing process and the mixing process, avoiding situations such as low hardness, poor fragility, and severe transportation powder detachment of the preparation tablets. For example, when using the hot melt extrusion method to prepare the solid dispersion, when the crushing speed is 5000-5400 rpm or/and the number of sieves is 60-120 meshes, the prepared solid dispersion has a good particle size distribution, effectively improving the compressibility of the solid formulation process. When preparing the solid formulation, when the mixing time of the solid dispersion and medicinal additives is 20-40 min, the resulting mixture has good mixing uniformity and good compressibility. The hardness of the prepared preparation tablets is about 80-135 N. Therefore, by optimizing the crushing process parameters and mixing process control, the compressibility of a tablet obtained from the solid dispersion system can be effectively improved, thereby enhancing the medicinal property of a solid formulation.

The solid formulation of the present invention may contain compound A ranging from 2 mg to 1500 mg. Patients can generally be adults or children, and can also be treated by other mammals.

The solid formulation provided by the present invention is suitable for mucosal administration to patients, which can be administered to the mucosa for transmembrane absorption. For this reason, appropriate routes of administration include inhalation, as well as oral, nasal, and rectal administration. Specially preferably oral administration. Technicians can select tablets, capsules, or other preparation forms based on the route of administration. However, other routes of administration, such as extraintestinal route, cannot be ruled out. For example, the solid formulation according to the present invention can be tablets, capsules, granules, powders, etc.

The solid formulation of the present invention has a higher bioavailability compared to solid formulations obtained by other methods. In a specific embodiment of the present invention, the relative bioavailability of the solid formulation of compound A is more than 1000% of that of the micronized preparation, the relative bioavailability of the solid formulation of compound A is more than 600% of that of the ordinary solid dispersion preparation, and the absolute bioavailability of the solid formulation of compound A is more than 40% (see Experimental Example 4). The improvement of bioavailability helps to reduce the required dose of equivalent exposure dose observed using conventional preparations (such as ordinary micronized preparation IR tablets), which can reduce the effective therapeutic dose of a drug, improve drug efficacy, save drug cost, and reduce drug toxicity and side effects.

On the other hand, the present invention also provides an use of the solid dispersion as described earlier or the solid formulation in the preparation of a drug for prevention and/or treatment of protein tyrosine kinase disorder-related disease and/or tumor.

In a preferred embodiment of the present invention, the protein tyrosine kinase disorder-related disease and/or tumor includes but not limited to solid cancer, for example, lung cancer, gastric cancer, esophageal cancer, colon cancer, colorectal cancer, liver cancer, renal cell cancer, head and neck cancer, thyroid cancer, ovarian cancer, breast cancer, pancreatic cancer, prostate cancer, oral cancer, malignant glioma cancer, rhabdomyosarcoma or osteosarcoma.

Preferably, the disease and/or tumor is lung cancer, gastric cancer, liver cancer, renal cell cancer, ovarian cancer, breast cancer, pancreatic cancer, prostate cancer, or thyroid cancer.

More preferably, the disease and/or tumor is lung cancer, for example, especially non-small cell lung cancer (NSCLC).

On the other hand, the present invention also provides an use of the solid dispersion as described earlier or the solid formulation in the preparation of a C-Met inhibitor.

The C-Met inhibitor is used to prepare a drug for the prevention or treatment of abnormal cell proliferation, morphological change, and hyperkinesia related to protein tyrosine kinase disorder in an organism, as well as diseases related to angiogenesis or cancer metastasis, for example, for the treatment or prevention of tumor growth and metastasis.

On the other hand, the present invention also provides a method for preventing and/or treating protein tyrosine kinase disorder-related disease and/or tumor, including administering an effective amount of the solid dispersion as described earlier or the solid formulation.

On the other hand, the present invention provides a kit of parts containing the solid dispersion as described earlier or the solid formulation.

The term "treatment" used herein includes administering a combination of the present invention to individuals in need to achieve the goals of a disease or condition or its symptoms (for example, cancer), including but not limited to relief, cure, symptom relief, symptom reduction, prolonged survival, and delayed progression; in terms of cancer, the treatment includes inhibiting the growth of solid tumors, reducing tumor volume, preventing metastatic spread of tumors, and preventing the growth or development of small metastases. "Delayed progression" refers to administering the combination to patients in the pre-cancerous stage or early stages of the cancer to be treated, and patients where the corresponding pre-form of the cancer has been diagnosed and/or diagnosed with a possible progression of the corresponding cancer.

The term "prevention" used herein includes the inhibition or postponement of the occurrence or frequency of a disease or condition or its symptoms (such as cancer), which typically refers to drug administration before the onset of the disease or symptoms, especially in high-risk individuals. "Prevention" also includes preventing the occurrence or recurrence of cancer.

The term "effective dose" used herein refers to the amount of active agent disclosed herein used (for example, therapeutic effective dose, especially combination therapy effective dose): (i) to treat a specific disease, (ii) to weaken, improve, or eliminate one or more symptoms of a specific disease, or (iii) to prevent or delay the onset of one or more symptoms of a specific disease described herein. As for cancer, the effective therapeutic dose of active agents can reduce the number of cancer cells; reduce tumor size; inhibit (i.e., to a certain extent, slow down and preferably stop) of cancer cell infiltration into surrounding organs; inhibit (i.e., to a certain extent, slow down and preferably stop) of tumor metastasis; to a certain extent, inhibit tumor growth; and/or to a certain extent, alleviate one or more symptoms related to cancer.

The term "individual" or "patient" used herein refers to both mammals and non-mammals. Mammals refer to any member of mammals, including but not limited to: humans; non-human primates, such as cows, horses, sheep, pigs, rabbits, dogs, and cats. "Individual" is not limited to a specific age or gender. Preferably, the individual or patient is a human.

The term "pharmaceutically acceptable" used in the present invention refers to nontoxic, biologically tolerable, and suitable for individual administration.

The "pharmaceutically acceptable salt" used in the present invention refers to the nontoxic, biologically tolerable acid addition salt or base addition salt of compound A suitable for individual administration, including but not limited to: an acid addition salt formed by compound A with an inorganic acid, for example, hydrochloride, hydrobromide, carbonate, bicarbonate, phosphate, sulfate, sulfite, nitrate, etc.; and an acid addition salt formed by compound A with an organic acid, for example, formate, acetate, malate, maleate, fumarate, tartrate, succinate, citrate, lactate, methanesulfonate, p-toluenesulfonate, 2-hydroxyethanesulfonate, benzoate, salicylate, stearate, and a salt formed by compound A with chain alkane dicarboxylic acid of formula HOOC-(CH₂)ₙ-COOH (wherein n is 0-4), etc. "Pharmaceutically acceptable salt" also includes a base addition salt formed by compound A with pharmaceutically acceptable cations such as sodium, potassium, calcium, aluminum, lithium, and ammonium.

The term "polymer" used herein refers to a macromolecule composed of repeating structural units connected by covalent bonds. This term includes linear and branched polymers, cyclic polymer such as cyclooligosaccharide (including cyclodextrin), homopolymer, and copolymer, whether from natural, synthetic, or semi-synthetic sources.

The term "matrix polymer" used herein refers to a material that exhibits low moisture absorption and high softening temperature, including a polymer or a blend of two or more polymers.

The "high softening temperature" used herein refers to the glass transition temperature (Tg) or melting point (Tm) > 100°C of a material measured by differential scanning calorimetry (DSC), wherein Tg is a measure suitable for a polymer in amorphous state or form, while Tm is a measure suitable for a polymer in crystalline state or form.

The term "surfactant" used herein refers to a medicinal surfactant.

The term "solid dispersion" used herein refers to a system that disperses a compound in an excipient carrier. In terms of the drug state in the system, a solid dispersion can comprise a composition wherein a drug is dispersed in discrete domains of crystalline or amorphous drugs or as independent molecules within an excipient carrier. In terms of the entire drug excipient complex, solid dispersions can be relatively large solid substances, such as pellets, tablets, films, or bundles; or they can exist as free-flowing powders composed of primary particles at the micrometer or nanometer level or their aggregates. In the present invention, the definition of the solid dispersion does not include a physical mixture from dry or wet mixing or dry blending operations, as well as a simple mixture of a compound crystal and other excipients.

The term "AUC" used herein refers to the area under the drug time curve, using its conventional meaning, that is, the area under the plasma concentration-time curve from 0 to 24 hours. AUC has a unit of concentration multiplied by time. Once the experimental concentration-time point is determined, AUC can be conveniently calculated, for example, through a computer program or through the trapezoidal method.

On the basis of not violating common knowledge in the art, the above preferred conditions can be arbitrarily combined to obtain the preferred examples of the present invention.

The reagents and raw materials used in the present invention are commercially available.

The positive progressive effect of the present invention lies in that:

The solid dispersion of the present invention can significantly improve the solubility and dissolution stability of compound A, prolong the oversaturation maintenance time of the drug, thereby improve the bioavailability of the solid formulation of compound A. The solid formulation of the present invention has high bioavailability. The high bioavailability reduces the required dose for the equivalent exposure dose observed in conventional preparations, which can reduce the effective therapeutic dose of a drug, improve drug efficacy, save drug cost, and reduce drug toxicity and side effects.

The present invention also effectively controls the decomposition of components in the dispersion by improving the process of preparing solid dispersion, especially the extrusion process, thereby reducing the impurity content of the dispersion. The present invention also greatly improves the compressibility of the tablets obtained from the solid dispersion system by optimizing the solid dispersion crushing process and the mixing process of the solid dispersion powder, avoiding situations such as low tablet hardness, poor fragility, and severe transportation powder detachment.

### BRIEF DESCRIPTION OF THE DRAWINGS

FIG. 1 shows the particle size diagram of the solid dispersion prepared in Example 1 of the present invention after being diluted with 5% SDS-simulated intestinal fluid;
FIG. 2 shows the comparison diagram (n = 6) of in vitro dissolution of various solid formulations prepared in Examples 5, 6, 7 and Comparative Examples 1 and 3 of the present invention;
FIG. 3 is a comparison diagram (n=6) of in vitro dissolution after 6 months of placement of the solid formulation prepared in Example 5 of the present invention under accelerated conditions and in vitro dissolution at 0 month;
FIG. 4 is a comparison diagram (n=6) of in vitro dissolution after 6 months of placement of the solid formulation prepared in Example 6 of the present invention under accelerated conditions and in vitro dissolution at 0 month;
FIG. 5 is a comparison diagram (n=6) of in vitro dissolution after 6 months of placement of the solid formulation prepared in Example 7 of the present invention under accelerated conditions and in vitro dissolution at 0 month;
FIG. 6 shows the dissolution stability investigation diagram of in vitro dissolution of various solid formulations prepared in Examples 5, 6, 7 and Comparative Examples 1 and 3 of the present invention.

### DETAILED DESCRIPTION OF EMBODIMENTS

The present invention is further explained through examples, but it is not limited to the scope of the examples. The experimental methods in the following examples that do not specify specific conditions shall be selected according to conventional methods and conditions, or according to the product manual.

In the present invention, the source and trade name of the reagents and equipment used are all indicated at the time of first appearance. If there are no special instructions, the same reagents used thereafter are the same as those first indicated. Conventional unmarked reagents are purchased from Sinopharm Chemical Reagent Co., Ltd. Among them, compound A was synthesized by Shanghai Institute of Materia Medica according to the method disclosed in CN104230922A.

Experimental animal: beagle dog, male, weighing 8-10 kg. The source is the Experimental Animal Center of Shanghai Institute of Materia Medica. The test animals were subjected to adaptive feeding at the experimental site for 3-7 days prior to the experimental day.

### Example 1

| **Ingredients** | **Parts by weight** |
|---|---|
| Compound A | 10.0 |
| Hydroxypropyl methylcellulose phthalate | 50.0 |
| Copovidone | 25.0 |
| Polyoxyl-40-stearate | 5.0 |
| Sodium dodecyl sulfate | 4.0 |
| Colloidal silica | 1.0 |

Preparation method: hydroxypropyl methylcellulose phthalate (50.0 parts by weight) (Shin-Etsu Chemical Co., Ltd., Japan, HP-55), copovidone (25.0 parts by weight) (PVP/VA64, BASF), polyoxyl-40-stearate (5.0 parts by weight) (Hunan Kang Pharmaceutical Limited by Share Ltd., S40), sodium dodecyl sulfate (4.0 parts by weight) (BASF) were mixed with compound A (10.0 parts by weight) and colloidal silica (1.0 parts by weight) (EVONIK, Aerosil), and then the powdered mixture was loaded into a twin-screw extruder (screw diameter 11 mm, Thermo Scientific) with an extrusion speed of 100 rpm and a temperature of 190°C, the mixture was extruded in a strip shape through the screw; and the extruded strip material was crushed and sieved through a 60 mesh sieve to obtain a solid dispersion 1 containing compound A.

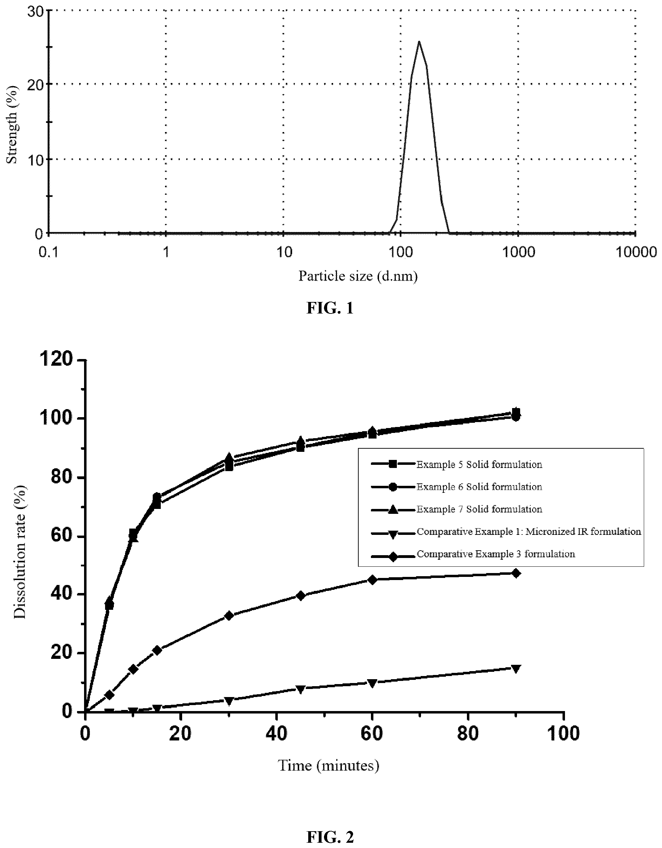

The solid dispersion 1 powder was added to 5% sodium dodecyl sulfate (SDS) and pH 6.8 simulated intestinal fluid (containing 6.8 g of potassium dihydrogen phosphate and 0.944 g of sodium hydroxide per liter of water), dissolved to determine the particle size of the formed polymer micelles (Zetasizer Nano ZS laser particle size analyzer, Malvern Instruments Ltd., UK). The average particle size of this product was measured to be 182.3 nm (FIG. 1).

The solubility of compound A was measured in a pH 6.8 simulated intestinal fluid containing different concentrations of SDS surfactants (1%, 3%, 5%) using solid dispersion 1 and compound A active pharmaceutical ingredient powder (shaken at 100 rpm for 6 h at 37°C). The solid dispersion group was sampled at 3 h and 6 h, and the measurement results are shown in Table 1. The test results show that the solid dispersion 1 prepared by the present invention can significantly improve the solubility of active pharmaceutical ingredient compound A, and the solid dispersion 1 can still maintain good solubility at 6 h without crystallization.

**Table 1 Solubility of compound A active pharmaceutical ingredient and solid dispersion 1**

| Solvent | Solubility (µg/mL) | | |
|---|---|---|---|
| | Compound A active pharmaceutical ingredient | Solid dispersion 1, 3 h | Solid dispersion 1, 6 h |
| 1% SDS-pH6.8 simulated intestinal fluid¹ | 21.4 | 157.6 | 153.9 |
| 3% SDS-pH6.8 simulated intestinal fluid² | 43.9 | 467.9 | 456.7 |
| 5% SDS-pH6.8 simulated intestinal fluid³ | 57.6 | 887.6 | 873.2 |

| | | | |
|---|---|---|---|
| Note: ¹each liter of water contained 10 g of sodium dodecyl sulfate, 6.8 g of potassium dihydrogen phosphate, and 0.944 g of sodium hydroxide; ²Each liter of water contains 30 g of sodium dodecyl sulfate, 6.8 g of potassium dihydrogen phosphate, and 0.944 g of sodium hydroxide; ³Each liter of water contains 50 g of sodium dodecyl sulfate, 6.8 g of potassium dihydrogen phosphate, and 0.944 g of sodium hydroxide. | | | |

After placing the solid dispersion 1 powder under accelerated conditions (40°C ± 2°C, 75% ± 5% RH) for 6 months, the solubility was measured (37°C, shaking at 100 rpm for 6 hours). The solubility was respectively 153.4 µg/mL, 449.6 µg/mL and 875.3 µg/mL in above 1%, 3% and 5% SDS-pH 6.8 simulated intestinal fluid. After being placed under accelerated conditions for 6 months, the solid dispersion 1 of the present invention still had a good solubilizing effect on compound A.

The above solubility measurement results indicate that the solid dispersion 1 prepared in this example has not crystallized, which can effectively prevent drug precipitation; and the oversaturation stability and long-term storage stability are good, which can prolong the oversaturation maintenance time of drugs and ensure effective absorption of drugs in the body.

### Example 2

| **Ingredients** | **Parts by weight** |
|---|---|
| Compound A | 15.0 |
| Hydroxypropyl methylcellulose phthalate | 55.0 |
| Copovidone | 20.0 |
| Glycerol monostearate | 6.0 |
| Sodium dodecyl sulfate | 6.0 |
| Colloidal silica | 1.0 |

Preparation method: hydroxypropyl methylcellulose phthalate (55.0 parts by weight) (Shin-Etsu Chemical Co., Ltd., Japan, HP-50), copovidone (20.0 parts by weight) (PVP/VA64, BASF), glycerol monostearate (6.0 parts by weight) (Hunan Kang Pharmaceutical Limited by Share Ltd.), sodium dodecyl sulfate (6.0 parts by weight) were mixed with compound A (15.0 parts by weight) and colloidal silica (1.0 parts by weight), and then the powdered mixture was loaded into a twin-screw extruder (screw diameter 11 mm) with an extrusion speed of 150 rpm and a temperature of 200°C, the mixture was extruded in a strip shape through the screw; and the extruded strip material was crushed and sieved through a 100 mesh sieve to obtain a solid dispersion 2 containing compound A.

The solubility of compound A of the solid dispersion 2 was measured by shaking at 37°C and 100 rpm for 6 h in a pH 6.8 simulated intestinal fluid containing different concentrations of SDS surfactants (1%, 3%, 5%). The solubility of solid dispersion 2 containing compound A in 1% SDS-pH 6.8 simulated intestinal fluid was determined to be 115.70 µg/mL; the solubility in the above 3% SDS-pH 6.8 simulated intestinal fluid was 424.5 µg/mL; and the solubility in the above 5% SDS-pH 6.8 simulated intestinal fluid was 723.1 µg/mL. The test results show that solid dispersion 2 containing compound A can significantly improve the solubility of compound A.

### Example 3

| **Ingredients** | **Parts by weight** |
|---|---|
| Compound A | 12.0 |
| Hydroxypropyl methylcellulose acetate succinate | 50.0 |
| Povidone | 15.0 |
| Sodium dodecyl sulfate | 4.0 |
| Glycerol monostearate | 12.0 |
| Colloidal silica | 2.0 |

Preparation method: hydroxypropyl methylcellulose acetate succinate (50.0 parts by weight) (Shin-Etsu Chemical Co., Ltd., Japan, model: HF), povidone (15.0 parts by weight) (PVP.K12, BASF), sodium dodecyl sulfate (4.0 parts by weight) (BASF), glycerol monostearate (12.0 parts by weight) (Hunan Kang Pharmaceutical Limited by Share Ltd.) were mixed with compound A (12.0 parts by weight) and colloidal silica (2.0 parts by weight), and then the powdered mixture was loaded into a twin-screw extruder (screw diameter 11 mm) with an extrusion speed of 100 rpm and a temperature of 180°C, the mixture was extruded in a strip shape through the screw; and the extruded strip material was crushed and sieved through a 90 mesh sieve to obtain a solid dispersion 3 containing compound A.

The solubility of compound A of the solid dispersion 3 was measured by shaking at 37°C and 100 rpm for 6 hours in a 5% SDS-pH 6.8 simulated intestinal fluid. The solubility of the solid dispersion 3 containing compound A was determined to be 815.5 µg/mL in a 5% SDS-pH 6.8 simulated intestinal fluid. The test results show that solid dispersion 3 containing compound A can significantly improve the solubility of compound A and is stable.

### Example 4

| **Ingredients** | **Parts by weight** |
|---|---|
| Compound A | 8.0 |
| Hydroxypropyl methylcellulose phthalate | 60.0 |
| Hydroxypropyl methylcellulose | 15.0 |
| Sodium dodecyl sulfate | 2.0 |
| Polyoxyl-40-stearate | 8.0 |
| Colloidal silica | 2.0 |

Preparation method: hydroxypropyl methylcellulose phthalate (60.0 parts by weight) (model: HP-50), hydroxypropyl methylcellulose (15.0 parts by weight) (HPMC HME 15 LV, DuPont, USA), sodium dodecyl sulfate (2.0 parts by weight), polyoxyl-40-stearate (8.0 parts by weight), colloidal silica (2.0 parts by weight) and compound A (8.0 parts by weight) were dissolved in a mixed solvent of acetone/dichloromethane (volume ratio 2:1), and then the solvent was evaporated at 30°C using a rotary evaporator. The resulting substance was dried in a 40°C vacuum drying oven for more than 12 h to remove residual organic solvents. The obtained solid material was crushed and sieved through a 60 mesh sieve to obtain a solid dispersion 4 containing compound A.

The solubility of compound A of the solid dispersion 4 was measured by shaking at 37°C and 100 rpm for 3 h and 6 h in a 5% SDS-pH 6.8 simulated intestinal fluid. The solubility of the solid dispersion 4 containing compound A was determined to be 765.5 µg/mL and 715.6 µg/mL in a 5% SDS-pH 6.8 simulated intestinal fluid for 3 h and 6 h. The test results show that solid dispersion 4 containing compound A can significantly improve the solubility of compound A and has a longer oversaturation maintenance time, which is conducive to drug absorption.

### Example 5

The solid dispersion 1 (95.0 parts by weight) obtained from Example 1 was mixed uniformly with copovidone (17.4 parts by weight) (PVP / VA64, BASF), crospovidone (3.6 parts by weight) (International Specialty Alloys Inc), and sodium stearyl fumarate (1.0 parts by weight) (German JRS Group Pharmaceutical Accessories Inc), and pressed into 585.0 mg tablets using a single punch tablet press. Then the tablets were placed in a coating pot and coated with an aqueous dispersion for film coating (Opadry, Shanghai Colorcon Coating Technology Co., Ltd) at a temperature of 60°C to obtain a solid formulation 1 containing compound A. This solid formulation was a tablet.

### Example 6

The solid dispersion 2 (103.0 parts by weight) obtained from Example 2 was mixed uniformly with microcrystalline cellulose (13.2 parts by weight) (Taiwan Mingtai Chemical Co., Ltd.), pre-gelatinized starch (8.0 parts by weight) (Shanghai Colorcon Coating Technology Co., Ltd), low substituted hydroxypropyl cellulose (4.8 parts by weight) (Shin-Etsu Chemical Co., Ltd., Japan), and magnesium stearate (1.0 parts by weight) (Anhui Shanhe Pharmaceutical Co., Ltd.), and a filling capsule filling machine was used to fill 0# capsules with 260 mg/capsule to obtain a solid formulation 2 containing compound A. This solid formulation is a capsule preparation.

### Example 7

The solid dispersion 3 (95.0 parts by weight) obtained from Example 3 was mixed uniformly with lactose (12.0 parts by weight) (made by DFE Pharma in the Netherlands), croscarmellose sodium (4.0 parts by weight), and sodium stearyl fumarate (1.0 parts by weight), and pressed into 467 mg tablets using a single punch tablet press. Then the tablets were placed in a coating pot and coated with an aqueous dispersion for film coating (Opadry, Shanghai Colorcon Coating Technology Co., Ltd) at a temperature of 60°C to obtain a solid formulation 3 containing compound A. This solid formulation was a tablet.

### Example 8

| **Ingredients** | **Parts by weight** |
|---|---|
| Compound A | 10.0 |
| Hydroxypropyl methylcellulose phthalate | 45.0 |
| Copovidone | 10.0 |
| Polyoxyl-40-stearate | 5.0 |
| Colloidal silica | 0.5 |

Preparation method: hydroxypropyl methylcellulose phthalate (45.0 parts by weight) (Shin-Etsu Chemical Co., Ltd., Japan), copovidone (10.0 parts by weight) (PVP/VA64, BASF), polyoxyl-40-stearate (5.0 parts by weight) (Croda Singapore Pte Ltd), compound A (10.0 parts by weight) and colloidal silica (0.5 parts by weight) (JRS) were pre-treated and mixed uniformly to obtain a powdered mixture. Then, the powdered mixture was loaded into a twin-screw extruder (with a screw diameter of 18 mm, LEISTRITZ) with an extrusion speed of 100-240 rpm and a temperature of 160-200°C. The feeding speed was 50-70 rpm, and the mixture was extruded in a strip shape through the screw; the extruded material was cooled by rapid cold pressing roller extrusion; the hot melt extruded strip was added into a hammer crusher for crushing treatment to obtain a solid dispersion 5 containing compound A.

In this example, for the obtained powdered mixture, different solid dispersions containing compound A were obtained through melt extrusion equipment with different process parameters; according to DSC and XRPD tests, these dispersions were all amorphous. In addition, the degradation product of phthalic acid in the solid dispersion during the preparation process was also detected, and the results are shown in Table 2 below.

**Table 2 Research results on different process parameters of hot melt extrusion**

| Process parameter | Paramete r 1 | Paramete r 2 | Paramete r 3 | Paramete r 4 | Paramete r 5 | Paramete r 6 | Paramete r 7 |
|---|---|---|---|---|---|---|---|
| Sleeve temperatur e (°C) | 150 | 160 | 160 | 170 | 180 | 180 | 180 |
| Screw speed (rpm) | 210 | 210 | 240 | 210 | 210 | 180 | 50 |
| Feeding speed (rpm) | 60 | 60 | 70 | 60 | 60 | 50 | 25 |
| Phthalic acid (wt%)¹ | 2.8 | 3.0 | 3.0 | 3.5 | 4.5 | 4.7 | 4.8 |

| | | | | | | | |
|---|---|---|---|---|---|---|---|
| Note: 'the content of phthalic acid was calculated based on the total components of the solid dispersion. | | | | | | | |

The results show that as the hot melt extrusion temperature increases, the degradation level of phthalic acid increases, and as the screw speed and feeding speed decrease, the degradation level of phthalic acid increases. By optimizing process parameters, it was possible to effectively avoid the growth of impurity phthalic acid caused by temperature rise and residence time increase.

In this example, the crushing process of solid dispersion was also studied. The study found that when the crushing speed was 5000-5400 rpm, or/or the number of sieves after crushing was 60-120 meshes, the prepared solid dispersion had a good particle size distribution (such as D90 < 200 µm), effectively improving the compressibility of the solid formulation process.

### Example 9

The solid dispersion 5 (75.0 parts by weight) obtained from Example 8 were mixed with copovidone (16.0 parts by weight) (PVP/VA64, BASF), crospovidone (20.5 parts by weight, ASHLAND), and citric acid (2.0 parts by weight, Merck) in a mixing bucket, at a mixing speed of 10 rpm and a mixing time of 20-40 min. Sodium dodecyl sulfate (3.0 parts by weight, BASF) and sodium stearyl fumarate (0.6 parts by weight, JRS) were added for lubrication, at a mixing speed of 10 rpm and a mixing time of 3-10 min to obtain a uniformly mixed total mixed powder. A Fette tablet press was used to press the tablets into 600 mg to prepare the corresponding preparation 4.

In this example, different mixing processes were studied when mixing and pressing the solid dispersion 5 to investigate the compressibility of the tablets during preparation. The results show that when the mixing time of the solid dispersion and medicinal additives is 20-40 min, the resulting total mixed powder obtained has good mixing uniformity, and the compressibility of the total mixed powder is good. The tablet hardness is about 80-135 N. In addition, when adding medicinal additives for mixing, especially when adding a surfactant and a lubricant (if any), the compressibility of the total mixed powder obtained by controlling the lubrication mixing time within 10 min was better. When the lubrication mixing time was too long, there was a problem that affected the compressibility of the powder; and the obtained tablet had a hardness of 50-70 N, which in turn affected its fragility.

### Example 10

| **Ingredients** | **Parts by weight** |
|---|---|
| Compound A | 10.0 |
| Hydroxypropyl methylcellulose phthalate | 35.0 |
| Copovidone | 5.0 |
| Polyoxyl-40-stearate | 5.0 |
| Colloidal silica | 0.5 |

Preparation method: hydroxypropyl methylcellulose phthalate (35.0 parts by weight) (HP-55, Shin-Etsu Chemical Co., Ltd., Japan), copovidone (5.0 parts by weight) (PVP/VA64, BASF), polyoxyl-40-stearate (5.0 parts by weight) (Croda Singapore Pte Ltd) were mixed with compound A (10.0 parts by weight) and colloidal silica (0.5 parts by weight, EVONIK) to obtain a powdered mixture. Then different preparation methods were used to obtain the corresponding solid formulation for the mixture.

Preparation method 1): the powdered mixture was dissolved in a mixed solvent of dichloromethane/methanol (volume ratio 10:1), and after dissolution, the solvent was evaporated at 40°C using a rotary evaporator. The sample was transferred to a vacuum drying oven (40°C, vacuum degree 0.9 bar) overnight (for more than 12 h) to remove residual organic solvents. The obtained dry material was ground and crushed, and sieved through an 80 mesh sieve for later use to obtain a solid dispersion 6 powder containing compound A.

Preparation method 2): the powdered mixture was loaded into a twin-screw extruder with an extrusion speed of 120 rpm and a temperature of 175°C, the mixture was extruded in a strip shape through the screw; and the extruded strip material was crushed and sieved through a 80 mesh sieve to obtain a solid dispersion 7 powder containing compound A.

The dissolution behavior of the dispersion powder and X-ray diffraction were used to investigate the solid dispersions prepared by the two processes. It was found that there was no significant difference in solubility between the two processes, and their ability to increase drug solubility was the same.

Solid dispersion 6 (55.5 parts by weight) and solid dispersion 7 (55.5 parts by weight) were mixed with copovidone (18.5.0 parts by weight) (PVP/VA64, BASF), crospovidone (20.5 parts by weight, ASHLAND), and citric acid (2.0 parts by weight, Merck) in a mixing bucket, at a mixing speed of 15 rpm and a mixing time of 20 min. Sodium dodecyl sulfate (3.0 parts by weight, BASF) and sodium stearyl fumarate (0.5 parts by weight, JRS) were added for lubrication, at a mixing speed of 15 rpm and a mixing time of 5 min to obtain a uniformly mixed total mixed powder. A tablet press was used to press to obtain the tablets of 500 mg, and then solid formulations T1 and T2 containing compound A were obtained, respectively.

**Table 3 Dissolution rates of solid formulations prepared by different methods**

| Time | | 0 min | 5 min | 10 min | 15 min | 30 min | 45 min | 60 min |
|---|---|---|---|---|---|---|---|---|
| Dissolution rate (%)¹ | Preparation T1 | 0 | 82.9 | 100.3 | 102.6 | 103.3 | 103.6 | 103.8 |
| | Preparation T2 | 0 | 66 | 79 | 87 | 98 | 101 | 101 |

| | | | | | | | | |
|---|---|---|---|---|---|---|---|---|
| Note: 'dissolution conditions: pH 6.8 phosphate buffer solution + 1% SDS, 75 rpm, paddle method. | | | | | | | | |

According to Table 3, the dissolution rate of the solid formulation T1 prepared by solvent evaporation method is faster than that of the solid formulation T2 prepared by hot melt extrusion method. However, after 30 minutes, both are completely released, and their dissolution behavior is basically the same with no significant difference. This indicates that the quality of the preparation prepared by solvent evaporation method and hot melt extrusion method is basically the same, indicating that there is no significant difference in the solubility of solid dispersions 6 and 7.

In addition, the bioavailability of solid formulations T1 and T2 was tested on beagle dogs (Beijing Marshall Biotechnology Co., Ltd., n=6), and the pharmacokinetic testing method was the same as in Example 5 of Experimental Example 4; the concentration of compound A in the sample was determined by LC-MS/MS, and the pharmacokinetic parameters of compound A after administration to beagle dogs were calculated using WinNonLin (8.3 version, Pharsight) using a non-compartment model.

**Table 4 Pharmacokinetic data of solid formulations containing compound A**

| PK parameter | Solid formulation T1 | Solid formulation T2 |
|---|---|---|
| AUC₍₀₋ₜ₎(h*ng/mL) | 1240 ± 674 | 2960 ± 1160 |
| Relative bioavailability F (%)¹ | 57.67 ± 25.00 | -- |

| | | |
|---|---|---|
| Note: 'relative bioavailability F = (AUC of solid formulation T1)/(AUC of solid formulation T2) x 100%, and the F value was taken as the average relative bioavailability of a single animal. | | |

The results show that the relative bioavailability of the solid formulation T1 prepared by solvent evaporation (rotary evaporation) is 57.67% of that of the solid formulation T2 prepared by melt extrusion method.

### Example 11

| **Ingredients** | **Parts by weight** |
|---|---|
| Compound A | 12.0 |
| Hydroxypropyl methylcellulose phthalate | 58.0 |
| Polyvinyl alcohol | 15.0 |
| Sodium dodecyl sulfate | 3.0 |
| Polyoxyl-40-stearate | 10.0 |
| Colloidal silica | 2.0 |

Preparation method: hydroxypropyl methylcellulose phthalate (58.0 parts by weight) (Shin-Etsu Chemical Co., Ltd., Japan), polyvinyl alcohol (15.0 parts by weight) (Merck), polyoxyl-40-stearate (10.0 parts by weight) (Nanjing Well Chemical Co., Ltd.) were mixed with compound A (12.0 parts by weight) and colloidal silica (2.0 parts by weight), and then the powdered mixture was loaded into a twin-screw extruder (screw diameter 16 mm) with an extrusion speed of 200 rpm and a temperature of 200°C, the mixture was extruded in a strip shape through the screw; and the extruded strip material was crushed and sieved through a 60 mesh sieve to obtain a solid dispersion 8 containing compound A.

The solubility of compound A of the solid dispersion 8 was measured by shaking at 37°C and 100 rpm for 6 hours in a 5% SDS-pH 6.8 simulated intestinal fluid. The solubility of the solid dispersion 8 containing compound A was determined to be 657.8 µg/mL in a 5% SDS-pH 6.8 simulated intestinal fluid for 6 hours. The test results show that solid dispersion 8 containing compound A can significantly improve the solubility of compound A.

### Comparative Examples

### Comparative Example 1

Compound A was micronized using an airflow crusher (MC JETMILL-50, JETPHARMA SOLUTIONS SA) to achieve an average particle size of approximately 20 µm (12.5 parts by weight), and mixed uniformly with hydroxypropyl methylcellulose phthalate (57.7 parts by weight), polyoxyl-40-stearate (4.7 parts by weight), sodium dodecyl sulfate (2.3 parts by weight), colloidal silica (0.9 parts by weight), copovidone (17.3 parts by weight) (PVP/VA64, BASF), crospovidone (3.6 parts by weight), and sodium stearyl fumarate (1.0 parts by weight), and pressed into a 400.0 mg micronized IR tablet using a single punch tablet press. Then the tablets were placed in a coating pot and coated with an aqueous dispersion for film coating (Opadry, Shanghai Colorcon Coating Technology Co., Ltd) at a temperature of 60°C to obtain a conventional solid formulation I containing compound A. This solid formulation was an ordinary micronized preparation.

### Comparative Example 2

Compound A (0.3 parts by weight) was mixed with propylene glycol (40.7 parts by weight) (Dow Chemical Company, USA) and polyoxyethylene castor oil (59.0 parts by weight) (BASF) in a suitable container. The mixture was stirred at a speed of 300 rpm at 70-110°C until compound A was completely dissolved, resulting in a liquid preparation containing compound A with a concentration of 3 mg/mL (1 wt%). The liquid preparation II containing compound A was obtained.

### Comparative Example 3

| Ingredients | Parts by weight |
|---|---|
| Compound A | 10.0 |
| Copovidone | 75.0 |
| Polyoxyl-40-stearate | 5.0 |
| Sodium dodecyl sulfate | 4.0 |
| Colloidal silica | 1.0 |

Preparation method: copovidone (75.0 parts by weight) (PVP.VA64, BASF), polyoxyl-40-stearate (5.0 parts by weight) (Hunan Kang Pharmaceutical Limited by Share Ltd., S40), sodium dodecyl sulfate (4.0 parts by weight) (BASF) were mixed with compound A (10.0 parts by weight) and colloidal silica (1.0 parts by weight) (EVONIK, Aerosil), and then the powdered mixture was loaded into a twin-screw extruder (screw diameter 11 mm, Thermo Scientific) with an extrusion speed of 100 rpm and a temperature of 170°C, the mixture was extruded in a strip shape through the screw; and the extruded strip material was crushed and sieved through a 60 mesh sieve to obtain a solid dispersion 10 containing compound A.

Solid dispersion 10 (95.0 parts by weight) was mixed uniformly with copovidone (17.4 parts by weight) (PVP VA64, BASF), crospovidone (3.6 parts by weight) (International Specialty Alloys Inc), and sodium stearyl fumarate (1.0 parts by weight) (German JRS Group Pharmaceutical Accessories Inc), and pressed into 585.0 mg tablets using a single punch tablet press. Then the tablets were placed in a coating pot and coated with an aqueous dispersion for film coating (Opadry, Shanghai Colorcon Coating Technology Co., Ltd) at a temperature of 60°C to obtain an ordinary solid dispersion preparation III containing compound A, which is a solid formulation prepared without an enteric high molecular polymer solid dispersion.

### Comparative Example 4

| **Ingredients** | **Parts by weight** |
|---|---|
| Compound A | 10.0 |
| Hydroxypropyl methylcellulose phthalate | 40.0 |
| Polyoxyl-40-stearate | 5.0 |
| Sodium dodecyl sulfate | 4.0 |
| Colloidal silica | 1.0 |

Preparation method: hydroxypropyl methylcellulose phthalate (40.0 parts by weight) (model: HP-50), polyoxyl-40-stearate (5.0 parts by weight) (Hunan Kang Pharmaceutical Limited by Share Ltd., S40), sodium dodecyl sulfate (4.0 parts by weight) (BASF) were mixed with compound A (10.0 parts by weight) and colloidal silica (1.0 parts by weight), and then the powdered mixture was loaded into a twin-screw extruder (screw diameter 11 mm) with an extrusion speed of 150 rpm and a temperature of 180°C, the mixture was extruded in a strip shape through the screw; a solid dispersion 11 containing compound A was obtained.

The solubility of compound A of the solid dispersion 11 was measured by shaking at 37°C and 100 rpm for 3 h and 6 h in a pH 6.8 simulated intestinal fluid containing SDS surfactants (1%). The solubility of solid dispersion 6 containing compound A in 1% SDS-pH 6.8 simulated intestinal fluid for 3 h was determined to be 53.7 µg/mL, 43.3 µg/mL for 6 h; the test results show that although the solid dispersion 6 containing compound A can slightly increase the solubility of compound A, but as time goes on, the solubility decreases, indicating drug precipitation and poor stability in oversaturated state, which is not conducive to drug absorption.

The solid dispersion 11 (300.0 parts by weight) was mixed uniformly with lactose (100.0 parts by weight), croscarmellose sodium (20.00 parts by weight), and sodium stearyl fumarate (2.00 parts by weight), and pressed into 422 mg tablets using a single punch tablet press to obtain the solid dispersion preparation IV containing compound A.

### Comparative Example 5

| **Ingredients** | **Parts by weight** |
|---|---|
| Compound A | 50.0 |
| Hydroxypropyl methylcellulose phthalate | 80.0 |
| Copovidone | 50.0 |
| Sodium dodecyl sulfate | 1.0 |
| Glycerol monostearate | 25.0 |
| Colloidal silica | 1.0 |

Preparation method: hydroxypropyl methylcellulose phthalate (80.0 parts by weight) (model: HP-50), copovidone (50.0 parts by weight) (PVP VA64, BASF), sodium dodecyl sulfate (1.0 parts by weight), glycerol monostearate (25.0 parts by weight), colloidal silica (1.0 part by weight) were mixed with compound A (50.0 parts by weight), and then the powdered mixture was loaded into a twin-screw extruder (screw diameter 11 mm) with an extrusion speed of 150 rpm and a temperature of 200°C, the mixture was extruded in a strip shape through the screw; and the obtained solid material was crushed and sieved through a 60 mesh sieve to obtain a solid dispersion 12 containing compound A (solid dispersion outside the weight fraction range of the present invention).

The solubility of compound A of the solid dispersion 12 was measured by shaking at 37°C and 100 rpm for 3 h and 6 h in a pH 6.8 simulated intestinal fluid containing SDS surfactants (1%). The solubility of solid dispersion 6 containing compound A in 1% SDS-pH 6.8 simulated intestinal fluid for 3 h was determined to be 31.3 µg/mL, 22.4 µg/mL for 6 h; the test results show that the solid dispersion 12 containing compound A has minimal effect on improving the solubility of compound A, but as time goes on, the solubility decreases, indicating drug precipitation and poor stability in oversaturated state, which is not conducive to improving drug absorption.

The solid dispersion 12 (207.0 parts by weight) was mixed uniformly with lactose (50.0 parts by weight), croscarmellose sodium (10.00 parts by weight), and sodium stearyl fumarate (2.00 parts by weight), and pressed into 269 mg tablets using a single punch tablet press to obtain the solid dispersion preparation V containing compound A.

### Experimental Examples

### Experimental Example 1

### Investigation of equilibrium solubility and permeability

Equilibrium solubility investigation: to investigate the solubility of compound A raw material in a series of solvents, the main solvents included water, pH 1.2 simulated gastric fluid (containing 2 g of potassium chloride and 7 mL of hydrochloric acid per liter of water), pH 4.5 phosphate buffer (containing 12.9 g of citric acid and 0.63 g of disodium hydrogen phosphate per liter of water), pH 6.8 simulated intestinal fluid. 0.1 g of compound A was taken, 100 mL of each of the above solvents were added, shaken at 100 rpm at 37°C for 24 h, and the supernatant was taken, centrifuged at 8000 rpm for 15 min to determine the concentration of compound A by HPLC. The solubility of compound A in different media is shown in Table 5.

**Table 5 Solubility of compound A in different solvents**

| Solvent | Equilibrium solubility (µg/mL), 24h |
|---|---|
| Water | 0.1 |
| pH 1.2 simulated gastric fluid | 0.5 |
| pH 4.5 phosphate buffer solution | 0.1 |
| pH 6.8 simulated intestinal fluid | 0.1 |

Permeability investigation: the permeability of compound A was evaluated using the Caco-2 cell model. The concentrations of compound A, positive control drug atenolol, propranolol, and digoxin were determined using the LC/MS/MS method. The apparent permeability coefficient (Papp) and the ratio of apparent permeability coefficient (Papp ratio = Papp_{(*B*→*A*)}/Papp_{(*A*→*B*})) were calculated, and the permeability of the compound and whether it was a substrate for P-gp were evaluated based on this. The results are shown in Table 6.

**Table 6 Results of permeability investigation of compound A in Caco-2 cell model (n = 3, ± SD)**

| Compound | Apparent permeability coefficient (*10⁻⁶ cm*/*s*) | | The ratio of apparent permeability |
|---|---|---|---|
| | *A*→*B* | *B*→*A* | |
| Atenolol | 0.41 ± 0.04 | 0.64 ± 0.07 | 1.57 |
| Propranolol | 13.7 ± 0.67 | 17.0 ± 0.32 | 1.24 |
| Digoxin | 0.08 ± 0.01 | 12.5 ± 0.90 | 165 |
| Compound A (2.00 µM) | 16.8 ± 3.36 | 20.3 ± 1.85 | 1.21 |
| Compound A (10.0 µM) | 4.81 ± 0.49 | 10.9 ± 0.35 | 2.27 |
| Compound A (50.0 µM) | 3.07 ± 0.09 | 1.96 ± 0.13 | 0.64 |

| | | | |
|---|---|---|---|
| Note: *A* = top, and *B* = outer side of base | | | |

According to Table 6, the solubility of compound A in media with different pH values is less than 1 µg/mL in the above solubility test results, indicating that it is an almost insoluble or insoluble drug; the permeability investigation results in Table 6 indicate that compound A has a high permeability characteristic. At high concentrations, the permeability coefficient may differ significantly from low concentrations due to the solubility of the drug. There is no significant efflux effect on Caco-2 cells within the concentration range of 2.00 µM and 50.0 µM.

### Experimental Example 2

### In vitro dissolution test investigation

The solid formulations 1, 2, and 3 prepared in Example 5, Example 6, and Example 7, as well as the solid formulations I and III prepared from Comparative Examples 1 and 3 were taken, and conducted dissolution tests according to the second method device (paddle method) of General Rule (0931) of Part Four of the 2015 edition of the Chinese Pharmacopoeia. Individual dosage units of each preparation were placed in 1000 mL of 5% SDS-pH 6.8 simulated intestinal fluid at a stirring speed of 50 rpm at 37°C. After 5, 10, 15, 30, 45, 60, and 90 min, 8 mL of the sample was taken out and administered the same volume of fluid. The sample taken out was diluted three times, UV visible spectrophotometry (General Rule 0401 of Part Four of the 2015 edition of the Chinese Pharmacopoeia) was used to measure the absorbance at a wavelength of 316 nm. The dissolution amount of the corresponding preparation was calculated and the dissolution curves were plotted (see FIG. 2).

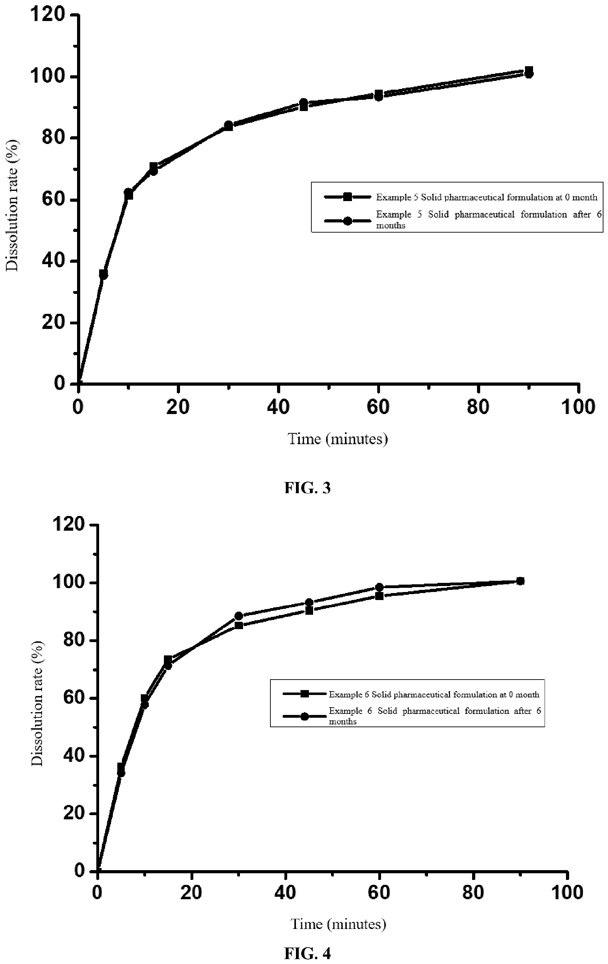

The solid formulations 1, 2, and 3 prepared in Examples 5, 6, and 7 were placed under accelerated conditions of 40 ± 2°C and 75% ± 5% RH for 6 months. The dissolution behavior of the drug was measured under the same conditions and the dissolution curves were plotted (see FIGS. 3, 4, and 5).

From FIGS. 2-5, it can be seen that the solid formulations 1, 2, and 3 prepared in Examples 5, 6, and 7 can significantly improve the dissolution rate and solubility of compound A compared to the IR conventional preparation (ordinary micronized preparation, Comparative Example 1) and the ordinary solid dispersion preparation III (solid formulation prepared by solid dispersion only containing non-enteric high molecular polymer, Comparative Example 3); moreover, the dissolution behavior of the solid formulation of the present invention does not significantly change after being placed under accelerated conditions, indicating good drug solubilization effect and stability.

### Experimental Example 3

### Dissolution stability investigation

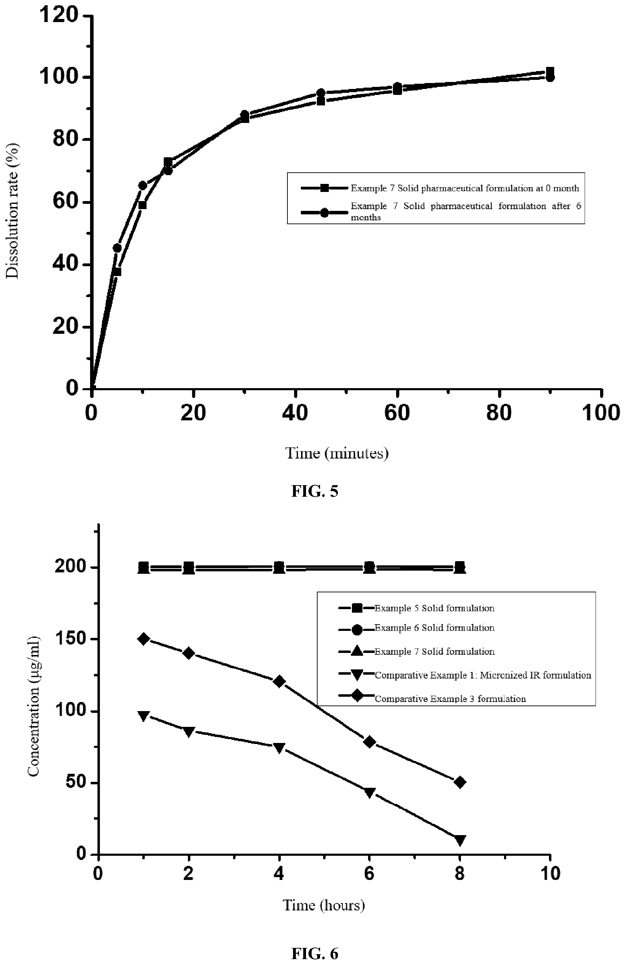

The preparations prepared in Example 5, Example 6 and Example 7, Comparative Example 1 and Comparative Example 3 were taken, and finely ground. Each dose unit of the preparations was weighed and placed in 250 mL of 3% SDS-pH 6.8 simulated intestinal fluid at 37°C and a stirring speed of 100 rpm. After 1 h, 2 h, 4 h, 6 h, and 8 h, the samples were taken out and centrifuged at 8000 rpm for 15 min to determine the concentration of compound A by HPLC and draw a time-concentration curve (see FIG. 6).

From FIG. 6, it can be seen that the solid formulations prepared in Example 5, Example 6, and Example 7 of the present invention have good dissolution stability and a stable supersaturated state within 8 hours, which is conducive to drug absorption. However, the IR conventional preparation (ordinary micronized preparation, Comparative Example 1) and ordinary solid dispersion preparation (solid formulation prepared by solid dispersion without non-enteric high molecular polymer, Comparative Example 3) showed a decrease in solubility after 2 hours, and the drug concentration continued to decrease with time, indicating drug precipitation, poor stability in oversaturated state, which is not conducive to drug absorption.

### Experimental Example 4

### Study on bioavailability in dogs

The preparations prepared in Example 5, Example 7, Comparative Example 1, Comparative Example 3, Comparative Example 4, and Comparative Example 5 were taken, and orally administered to full-bellied beagle dogs (n = 3, Experimental Animal Center of Shanghai Institute of Materia Medica). The preparation of Example 5, Example 7, Comparative Example 1, Comparative Example 3, Comparative Example 4, and Comparative Example 5 has a dosage of 50 mg per dog. Food was provided uniformly before the experiment, and medication was administered 30 min later. Throughout the entire experiment, water couldn't be avoided, and the cleaning period was 7 days. 0.5 mL of blood was taken from the veins of the limbs before administration (0 h) and at 0.25, 0.5, 1.0, 2.0, 4.0, 6.0, 8.0, 12, 24, 48, and 72 h after administration; placed in EDTA-K₂ anticoagulant test tube, centrifuged at 3500 rpm for 10 min, plasma was separated, and freezed in a -70°C freezer for testing.

The liquid preparation of Comparative Example 2 was taken and diluted 6 times with physiological saline, and administered intravenously to full-bellied beagle dogs (n = 3) at a dose of 1 mg/kg compound A (2 mL/kg). Food was provided uniformly before the experiment, and medication was administered 30 min later. Throughout the entire experiment, water couldn't be avoided. 0.5 mL of blood sample was taken before administration (0 h) and at 5 min, 0.25, 0.5, 1.0, 2.0, 4.0, 6.0, 8.0, 12, 24, 48, and 72 h after administration; placed in EDTA-K₂ anticoagulant test tube, centrifuged at 3500 rpm for 10 min, plasma was separated, and freezed in a -70°C freezer for testing.

The concentration of compound A in the sample was measured using LC-MS. The pharmacokinetic parameters of compound A after administration to beagle dogs were calculated using a non-compartmental model using Phoenix6.4 software (Pharsight, USA). The data summary is shown in Table 7.

**Table 7 Pharmacokinetic data of compound A (n = 3)**

| Preparation | T_{1/2}(h) | Cₘₐₓ(ng/mL) | AUC_{(0-∞)}(h*ng/mL) | Absolute bioavailability (%)¹ |
|---|---|---|---|---|
| Example 5 | 4.59 | 359 | 3610 | 52.7 |
| Example 7 | 5.21 | 301 | 2880 | 42.0 |
| Comparative Example 1 | 6.39 | 24 | 225 | 3.3 |
| Comparative Example 3 | 5.55 | 62 | 431 | 6.3 |
| Comparative Example 4 | 5.02 | 106 | 851 | 12.4 |
| Comparative Example 5 | 6.12 | 75 | 615 | 9.0 |
| Comparative Example 2 | 3.63 | / | 1370 | 100 |

| | | | | |
|---|---|---|---|---|
| Note: ¹The absolute bioavailability of the liquid preparation (Comparative Example 2) was 100%, and the bioavailability of other examples relative to Comparative Example 2 was tested. | | | | |

From the results, it can be seen that compared with the IR conventional preparation prepared in Comparative Example 1 (ordinary micronized preparation), the ordinary solid dispersion preparation prepared in Comparative Example 3 (solid formulation prepared by solid dispersion only containing non-enteric high molecular polymer), the solid dispersion preparation prepared in Comparative Example 4 (solid formulation prepared by solid dispersion only containing enteric high molecular polymer), and the solid dispersion preparation prepared in Comparative Example 5 (solid dispersion preparation outside the weight fraction range of the present invention), both the solid formulation 1 prepared in Example 5 of the present invention and the solid formulation 3 prepared in Example 7 of the present invention can significantly improve the bioavailability of compound A and have good in vivo absorption. In Comparative Example 1, Comparative Example 3, Comparative Example 4, and Comparative Example 5, the in vivo absorption is poor.

## Claims

1. A solid dispersion, **characterized by** comprising compound A and a pharmaceutically acceptable matrix polymer, wherein the pharmaceutically acceptable matrix polymer includes an enteric high molecular polymer and a non-enteric high molecular polymer, and the compound A is 1-{(6-[(1-methyl)-4-pyrazolyl]-imidazo[1,2-a]pyridine)-3-sulfonyl}-6-[(1-methyl)-4-pyrazolyl]-1-hydro-pyrazolo[4,3-b]pyridine, with a weight ratio of the compound A to the pharmaceutically acceptable matrix polymer of 1:3-1:35.

2. The solid dispersion of claim 1, **characterized in that** the enteric high molecular polymer is selected from one or more of hydroxypropyl methylcellulose phthalate, hydroxypropyl methylcellulose acetate succinate, polymethacrylate, polyvinyl acetate phthalate, cellulose acetate phthalate, and cellulose acetate succinate;
and/or, the non-enteric high molecular polymer is selected from one or more of polyvinyl caprolactam-polyvinyl acetate-polyethylene glycol graft copolymer, copovidone, povidone, polyvinyl alcohol, 2-hydroxy-β-cyclodextrin, hydroxypropyl methylcellulose, and hydroxypropyl cellulose;
and/or, the weight ratio of the enteric high molecular polymer to the non-enteric high molecular polymer is 2:1-10:1.

3. The solid dispersion of claim 1, **characterized in that** the solid dispersion meets one or more of the following conditions:
(i) the enteric high molecular polymer is hydroxypropyl methylcellulose phthalate and/or hydroxypropyl methylcellulose acetate succinate;
(ii) the non-enteric high molecular polymer is selected from one or more of copovidone, polyvinyl alcohol, povidone, and hydroxypropyl methylcellulose;
(iii) the solid dispersion also optionally comprises one or more of a flow aid, a plasticizer, and a surfactant;
(iv) the weight ratio of the enteric high molecular polymer to the non-enteric high molecular polymer is 2:1-6: 1;
and (v) the weight ratio of the compound A to the pharmaceutically acceptable matrix polymer is 1:4-1:25, preferably 1:5-1:15.

4. The solid dispersion of claim 3, **characterized in that** the solid dispersion meets one or more of the following conditions:
(i) the weight ratio of the compound A to the enteric high molecular polymer is 1:2-1:15, preferably 1:3-1:10;
(ii) the weight ratio of the compound A to the non-enteric high molecular polymer is 2:1 - 1:10, preferably 2:1-1:5;
(iii) the flow aid is selected from one or more of colloidal silica, animal fat, plant fat, and wax;
(iv) the weight ratio of the flow aid to the compound A is 1:1-1:100;
(v) the plasticizer is selected from one or more of acetyl tributyl citrate, acetyl triethyl citrate, benzyl benzoate, trichlorobutyl alcohol, dextrin, dibutyl phthalate, diethyl phthalate, dimethyl phthalate, glycerol, glyceride monostearate, polyoxyl-40-stearate, mannitol, mineral oil, lanolin alcohol, palmitic acid, polyethylene glycol, polyethylene glycol monostearate, polyvinyl alcohol acetate phthalate, propylene glycol, 2-pyrrolidone, sorbitol, stearic acid, triacetin, tributyl citrate, triethanolamine, and triethyl citrate;
(vi) the weight ratio of the plasticizer to the compound is 1:1-1:20;
(vii) the surfactant is selected from one or more of an anionic surfactant, a cationic surfactant, and a non-ionic surfactant; the anionic surfactant is preferably sodium dodecyl sulfate and/or docusate sodium; the cationic surfactant is preferably one or more of cetrimide, benzethonium chloride, cetylpyridinium chloride, and lauric acid; the non-ionic surfactant is preferably one or more of polyoxyethylene alkyl ether, polyoxyethylene sorbitan fatty acid ester, polyoxyethylene castor oil derivative, polyoxyethylene stearate, and polyoxyethylene polyoxypropylene ether block copolymer;
and (viii) the weight ratio of the surfactant to the compound A is 1:1-1:10.

5. A method for preparing the solid dispersion of any one of claims 1-4, **characterized by** including the following steps:
(1) mixing the various ingredients of the solid dispersion uniformly by melting or dissolution to obtain a uniform dispersion; and
(2) solidifying the uniform dispersion to obtain the solid dispersion.

6. The method for preparing the solid dispersion of claim 5, **characterized in that** the solidification is a melt extrusion method, and the method for preparing the solid dispersion meets one or more of the following conditions:
(i) in the melt extrusion method, a sleeve temperature of a melt extrusion equipment is 150-220°C;
(ii) in the melt extrusion method, a screw extrusion rotating speed of the melt extrusion equipment is 50-300 rpm;
(iii) in the melt extrusion method, the feeding speed is 10-100 rpm;
and (iv) the melt extrusion method includes:
(1a) mixing the various ingredients of the solid dispersion uniformly to obtain a powdered mixture;
and (2a) loading the powdered mixture into a hot melt extruder feeder, extruding, crushing, and screening to obtain the solid dispersion comprising the compound A.

7. A solid formulation, **characterized by** comprising the solid dispersion of any one of claims 1-4 and a medicinal additive; and preferably, the medicinal additive comprises one or more of a flow aid, an adhesive, a disintegrant, a filler, a lubricant, a colorant, a pH regulator, a surfactant, a lubricant, and a stabilizer.

8. The solid dispersion of any one of claims 1-4 or the solid formulation of claim 7, wherein, based on the total components of the solid dispersion, the content of phthalic acid is ≤ 6.0 wt%, preferably ≤ 4.8 wt%.

9. Use of the solid dispersion of any one of claims 1-4 or the solid formulation of claim 7 in preparation of a drug for the prevention and/or treatment of protein tyrosine kinase disorder-related disease and/or tumor.

10. The use of claim 9, wherein the disease and/or tumor comprises a solid cancer, for example, lung cancer, gastric cancer, esophageal cancer, colon cancer, colorectal cancer, liver cancer, renal cell cancer, head and neck cancer, thyroid cancer, ovarian cancer, breast cancer, pancreatic cancer, prostate cancer, oral cancer, malignant glioma cancer, rhabdomyosarcoma or osteosarcoma;
preferably, the disease and/or tumor is lung cancer, gastric cancer, liver cancer, renal cell cancer, ovarian cancer, breast cancer, pancreatic cancer, prostate cancer, or thyroid cancer;
and more preferably, the disease and/or tumor is lung cancer, for example, non-small cell lung cancer.

11. A method for preventing and/or treating protein tyrosine kinase disorder-related disease and/or tumor, including administering an effective amount of the solid dispersion of any one of claims 1-4 or the solid formulation of claim 7 to individuals in need.
